# FASCICULE DE BREVET EUROPEEN

(11) **EP 2 512 346 B1**
(45) Date de publication et mention de la délivrance du brevet: **06.08.2014**
(21) Numéro de dépôt: 10809278.4
(22) Date de dépôt: 15.12.2010
(51) Int. Cl.: A61B 6/06

(54) **APPAREIL DE RADIOLOGIE DENTAIRE FOURNISSANT UNE IMAGE CEPHALOMETRIQUE ET PROCEDE ASSOCIE**
ZAHNRÖNTGENGERÄT MIT ZEPHALOMETRISCHER BILDGEBUNG UND ZUGEHÖRIGES VERFAHREN
DENTAL X-RAY APPARATUS PROVIDING A CEPHALOMETRIC IMAGE AND RELATED METHOD

(30) Priorité: 16.12.2009 FR 0959083
(43) Date de publication de la demande: 24.10.2012
(73) Titulaire: Trophy, 77435 Marne La Vallée Cedex 2 (FR)
(72) Inventeur: LOUSTAUNEAU, Vincent, F-94120 Fontenay-sous-Bois (FR); BOTHOREL, Sylvie, F-77435 Marne La Vallée Cedex 2 (FR); INGLESE, Jean-Marc, F-77600 Bussy-Saint-Georges (FR)
(74) Mandataire: Wimmer, Hubert
(86) Numéro de dépôt international: PCT/FR2010/052752
(87) Numéro de publication internationale: WO 2011/080460

(56) Documents cités:
- WO-A1-2008/113715
- US-A- 4 603 427
- US-A- 5 970 112
- US-A1- 2003 235 265
- US-A1- 2004 202 283
- US-B1- 6 778 636

## Description

La présente invention concerne un appareil de radiologie dentaire et un procédé associé.

Dans le domaine de la radiologie dentaire, il est connu de réaliser des clichés céphalométriques de la tête d'un patient en vue de face et/ou de profil.

De tels clichés sont obtenus à partir d'un appareil de radiologie qui comprend un générateur de rayons X et un capteur de rayons X. Le capteur est placé en vis-à-vis du générateur et la tête du patient est installée entre le générateur et le capteur.

Le générateur émet un rayonnement sous la forme d'un faisceau conique de rayons X en direction de la tête du patient et le capteur reçoit le rayonnement ayant irradié la tête.

Ce rayonnement reçu permet d'obtenir une projection complète du crâne (tissus durs) du patient qui constitue un cliché céphalométrique.

Cette projection est obtenue par balayage de la tête du patient à partir d'un mouvement continu synchronisé entre une fente de collimation et le capteur par exemple réalisé sous la forme d'une barrette CCD disposée derrière la tête.

Un tel balayage dure environ 10 secondes.

Afin de diminuer l'effet de cône du faisceau de rayons X qui se traduit par des distorsions géométriques importantes (le côté du crâne situé le plus près du capteur est plus grand que le côté opposé du crâne), le capteur est placé à une distance suffisamment grande du générateur (par exemple de 1,60 m, voire davantage) et la tête du patient est placée bien plus près du capteur que du générateur.

A partir d'un ou de plusieurs clichés céphalométriques, (profil, face... ) un praticien, par exemple, un orthodontiste, établit un diagnostic faisant état de certains défauts à corriger chez le patient. Le praticien prend ensuite des mesures et/ou réalise des tracés afin de déterminer les corrections à apporter et le traitement approprié.

Pour pouvoir visualiser de façon prévisionnelle les effets au cours du temps des corrections et du traitement envisagés sur le visage du patient, il est également prévu de réaliser un ou plusieurs clichés photographiques du visage.

Ainsi, en pratique, le praticien prend un cliché photographique, par exemple suivant une vue de profil de la tête du patient, et en conserve uniquement les contours.

Ce cliché est ensuite superposé par logiciel au cliché céphalométrique réalisé suivant une vue de profil de la tête du patient.

A partir du moment où le praticien a déterminé les corrections et le traitement adaptés au patient, il peut simuler leurs effets sur le visage du patient et les visualiser.

Plus particulièrement, le logiciel dont dispose le praticien lui permet de visualiser, sur une première image correspondant à l'état actuel du patient, le cliché photographique représentant les tissus mous du crâne (nez, lèvres ...) superposé au cliché céphalométrique représentant les tissus durs (os, dents ...).

Le logiciel permet ensuite, par calcul, à partir des données sélectionnées par le praticien lorsqu'il a déterminé les corrections et le traitement appropriés, de déformer en conséquence le cliché céphalométrique afin de simuler les effets au cours du temps des corrections et du traitement sélectionnés sur les tissus durs.

Le cliché photographique est également déformé de façon correspondante par un algorithme de morphing.

Le logiciel permet enfin de visualiser une deuxième image superposant le cliché photographique et le cliché céphalométrique déformés et qui représente l'évolution de la tête du patient après corrections et traitement.

Ainsi, on peut contrôler de façon prévisionnelle l'évolution du traitement en visualisant les deux images simultanément.

Toutefois, pour que les clichés photographiques et céphalométriques soient superposables de façon satisfaisante, la demanderesse s'est aperçue que les clichés doivent être réalisés avec le même angle de prise de vue.

Par ailleurs, le générateur de rayons X est muni d'un collimateur dont les dimensions sont calculées pour s'adapter à une tête de dimensions moyennes.

Or, la demanderesse s'est aperçue que les têtes humaines présentent une grande dispersion dans leurs dimensions.

Ainsi, pour une personne possédant une tête dont les dimensions sont supérieures aux dimensions moyennes précitées, le ou les clichés céphalométriques représenteront un crâne tronqué.

Au contraire, le ou les clichés céphalométriques obtenus pour une tête dont les dimensions sont inférieures aux dimensions moyennes représenteront la totalité du crâne ainsi qu'une zone de contour inutile.

De ce fait, la personne aura reçue une surdose de rayonnement inutile.

La présente invention vise à remédier à au moins un des inconvénients précités en proposant un appareil de radiologie dentaire comprenant :
- un générateur de rayonnement X adapté à générer un faisceau de rayons X en direction d'une tête d'un patient,
- des moyens de collimation adaptés à conférer au faisceau de rayons X généré des dimensions données;
- un capteur disposé en vis-à-vis du générateur, recevant la projection radiologique du faisceau collimaté ayant irradié la tête du patient et fournissant une image céphalométrique de la tête du patient,
   caractérisé en ce que l'appareil comporte :
   - des moyens d'acquisition d'au moins une image photographique de la tête du patient,
   - des moyens d'asservissement des moyens de collimation en fonction de ladite au moins une image photographique afin que les dimensions du faisceau de rayons X collimaté soient ajustées aux dimensions de la tête du patient.

En acquérant une ou plusieurs images photographiques de la tête du patient et, plus particulièrement, de son profil et en collimatant le faisceau de rayons X de façon asservie à l'image ou aux images photographiques ainsi acquises, il est possible d'ajuster les dimensions du faisceau collimaté aux dimensions de la tête du patient et, plus particulièrement, aux dimensions de son profil.

Une image photographique ou photographie de la tête d'un patient capture les parties visibles de la tête et du visage et, notamment, les contours de la tête (en vue de face ou de profil). En général, une telle image est donc représentative des tissus mous de la tête du patient (nez, lèvres...). Une telle image ne capture pas les parties cachées de la tête et qui représentent notamment les tissus durs (os, dents...). Ces parties sont en effet capturées par le capteur radiologique qui reçoit un rayonnement X ayant irradié la tête du patient. Un tel capteur fournit une image céphalométrique de la tête du patient qui est donc différente de l'image photographique précitée.

On notera que les moyens d'acquisition d'image(s) photographique(s) sont distincts du capteur radiologique qui forme un moyen d'acquisition d'image(s) céphalométrique(s).

Selon une caractéristique, le capteur est un capteur surfacique à matrice de pixels présentant des dimensions qui englobent les dimensions de la projection radiologique du faisceau ayant irradié la tête du patient. L'acquisition de la projection radiologique est effectuée de façon instantanée.

Selon une caractéristique, le générateur de rayonnement X comprend un foyer d'émission des rayons X, les moyens d'acquisition d'au moins une image photographique étant positionnés le plus près possible du foyer.

En disposant de façon aussi proche que possible les moyens d'acquisition d'image du foyer d'émission des rayons X, on s'assure ainsi que les clichés photographiques et céphalométriques sont pris suivant la même angulation ou, en tout cas, suivant une angulation très proche, compte tenu de la distance entre le foyer d'émission des rayons X et la tête du patient qui est relativement éloignée.

Il convient en effet de noter que la distance entre les moyens d'acquisition d'au moins une image photographique et le foyer d'émission est petite comparée à la distance entre le foyer et la tête du patient.

Cette distance est par exemple dans un rapport de 1 à 15.

Selon une caractéristique, les moyens de collimation comprennent un collimateur à fente réglable.

Il est ainsi particulièrement simple d'asservir les moyens de collimation en fonction de l'image ou des images photographiques acquises en procédant au réglage de la fente de façon appropriée.

Selon une caractéristique, le collimateur à fente réglable comporte des moyens de réglage de l'allongement de la fente selon des directions perpendiculaires entre elles.

Selon une caractéristique, les moyens de réglage sont indépendants selon les directions, ce qui procure une grande souplesse au réglage.

Selon une caractéristique, la fente réglable est délimitée par quatre bords qui sont déplaçables de façon indépendante l'un de l'autre.

Selon une caractéristique, l'appareil comprend des moyens d'obtention du contour de la tête du patient à partir de ladite au moins une image photographique acquise.

Ce contour contient suffisamment d'informations pour permettre d'asservir les moyens de collimation.

Selon une caractéristique, les moyens d'asservissement sont adaptés à asservir les moyens de collimation en fonction des dimensions du contour de la tête du patient afin que les dimensions du faisceau de rayons X collimaté soient ajustées aux dimensions du contour de la tête du patient.

Les dimensions du faisceau collimaté, et notamment la largeur du faisceau à sa base qui est proche du foyer d'émission, peuvent être asservies au contour de la tête du patient ainsi obtenu.

Ceci permet d'ajuster les dimensions du faisceau collimaté aux dimensions du contour de la tête du patient.

L'invention a également pour objet, de façon correspondante, un procédé pour produire une image céphalométrique de la tête d'un patient comprenant les étapes suivantes :
- génération par un générateur de rayonnement X d'un faisceau de rayons X en direction de la tête d'un patient,
- collimation du faisceau de rayons X généré afin de lui conférer des dimensions données,
- réception par un capteur en vis-à-vis de la projection radiologique du faisceau collimaté ayant irradié la tête du patient,
- fourniture d'une image céphalométrique à partir de la projection radiologie reçue,
   caractérisé en ce que le procédé comporte en outre les étapes suivantes :
- acquisition d'au moins une image photographique de la tête du patient,
- asservissement de la collimation du faisceau de rayons X en fonction de ladite au moins une image photographique afin que les dimensions du faisceau de rayons X collimaté soient ajustées aux dimensions de la tête du patient.

Le procédé selon l'invention comporte les mêmes avantages que ceux décrits brièvement ci-dessus en référence à l'appareil de radiologie dentaire et ils ne seront donc pas répétés ici.

Selon une caractéristique, le capteur est un capteur surfacique à matrice de pixels présentant des dimensions qui englobent les dimensions de la projection radiologique du faisceau ayant irradié la tête du patient, l'acquisition de la projection radiologique étant effectuée de façon instantanée.

Selon une caractéristique, l'étape d'asservissement de la collimation du faisceau de rayons X en fonction de la dite au moins une image photographique comprend le réglage des dimensions du faisceau.

Selon une caractéristique, le générateur de rayonnement X comprend un foyer d'émission des rayons X, l'acquisition de ladite au moins une image photographique étant effectuée à partir d'un emplacement qui est le plus proche possible du foyer.

Selon une caractéristique, l'étape d'asservissement du faisceau de rayons X en fonction de ladite au moins une image photographique comprend le réglage des dimensions du faisceau.

Selon une caractéristique, le réglage des dimensions du faisceau comprend plus particulièrement le réglage de l'allongement d'une fente de collimation selon des directions perpendiculaires entre elles.

Selon une caractéristique, le procédé comprend une étape d'obtention du contour de la tête du patient à partir de ladite au moins une image photographique acquise.

Selon une caractéristique, l'asservissement de la collimation est effectué en fonction des dimensions du contour de la tête du patient afin que les dimensions du faisceau de rayons X collimaté soient ajustées aux dimensions du contour.

D'autres caractéristiques et avantages apparaîtront au cours de la description qui va suivre, donnée uniquement à titre d'exemple non limitatif et faite en référence aux dessins annexés, sur lesquels :
- la Figure 1 est une vue schématique générale d'un appareil de radiologie selon l'invention ;
- les Figures 2a et 2b illustrent respectivement des moyens de collimation utilisés dans l'appareil de la Figure 1 ;
- la Figure 2c illustre schématiquement une fente de collimation obtenue avec les moyens des Figures 2a et 2b ;
- la Figure 3a illustre de façon schématique en vue de face l'extrémité 16b de l'appareil de la Figure 1 ;
- la Figure 3b illustre de façon schématique l'agencement des moyens d'acquisition d'un cliché photographique, du foyer d'émission des rayons X, de la tête d'un patient et du capteur 20 ;
- la Figure 4 illustre un algorithme d'un procédé de production d'une image céphalométrique ;
- les Figures 5a et 5b illustrent schématiquement la projection radiologique d'un faisceau irradiant la tête d'un patient avec les bords d'un collimateur non ajusté ;
- les Figures 5c et 5d illustrent schématiquement la projection radiologique d'un faisceau irradiant la tête d'un patient avec les bords du collimateur ajusté ;
- la Figure 6 illustre de façon schématique la superposition des clichés céphalométriques et photographiques ;
- la Figure 7 illustre de façon schématique une vue agrandie d'une partie de l'image de la Figure 6 ;
- la Figure 8 illustre de façon schématique les tissus durs du cliché céphalométrique déformés par calcul.

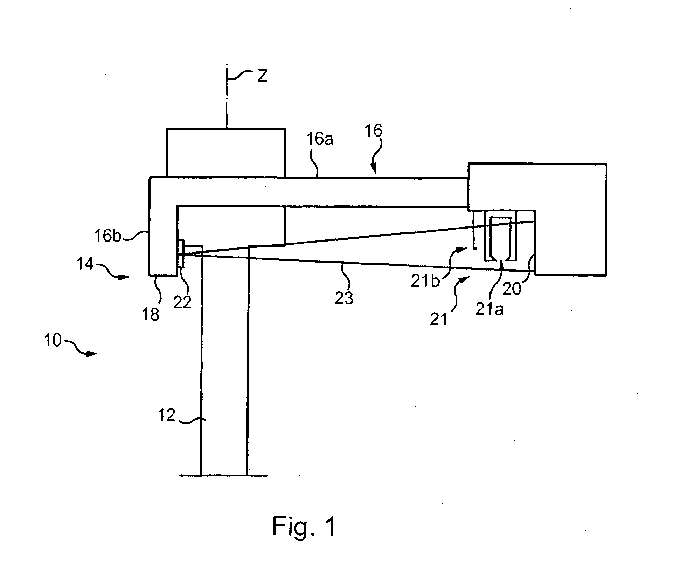

Comme représenté à la figure 1 et désigné par la référence générale notée 10, un appareil de radiologie dentaire selon l'invention est un appareil du type céphalométrique. Cet appareil permet de produire des images ou clichés céphalométriques de la tête d'un être humain. L'appareil comprend un bâti 12 fixe, par exemple une potence verticale alignée suivant l'axe Z, sur laquelle est montée une unité radiographique 14 qui va maintenant être décrite.

Cette unité comprend une structure 16 comportant une poutre horizontale 16a formant support qui comprend, à une extrémité, un bras vertical 16b descendant à partir de la poutre horizontale et, à l'extrémité opposée éloignée, un bras 16c qui est à la fois horizontal et vertical.

Une source ou générateur de rayons X 18 est montée de manière fixe sur le bras 16b, tandis qu'un capteur de rayons X 20 est monté sur le bras 16c éloigné qui permet de positionner le capteur à une grande distance du générateur, par exemple à 4 m de celui-ci.

Le générateur 18 et le capteur 20 sont ainsi disposés en vis-à-vis l'un de l'autre et sont dans une relation géométrique fixe l'un par rapport à l'autre.

La structure 16 qui sert de support au générateur 18 et au capteur 20 constitue le cceur de l'unité radiographique 14.

L'appareil de radiologie 10 comporte également, un dispositif de positionnement 21 fixé au bras 16c devant le capteur 20 et qui permet d'immobiliser la tête du patient pendant la prise de clichés radiographiques, lors du fonctionnement de l'appareil. La tête est ainsi intercalée entre le générateur 18 et le capteur 20. Plus particulièrement, le dispositif 21 comporte d'une part, une fourche verticale descendante 21a dont les deux branches possèdent des extrémités libres en regard destinées à être positionnées dans les oreilles du patient et, d'autre part, une tige verticale 21 b descendante, destinée à venir en contact avec le front du patient afin d'empêcher un mouvement avant-arrière de la tête.

Le générateur de rayonnement est équipé d'un support 22, agencé contre la face du générateur qui fait face au capteur 20 et dans laquelle est aménagée une ouverture pour la sortie des rayons X issus du générateur.

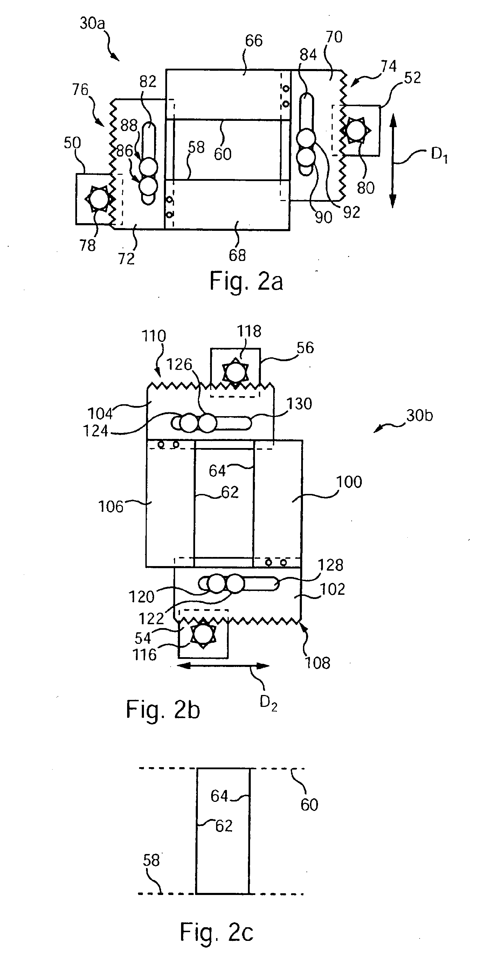

Le support est positionné devant cette fenêtre de sortie des rayons X et comporte des moyens de collimation qui seront décrits en référence aux figures 2a et 2b.

Le faisceau de rayons X collimaté a ainsi une forme de cône 23 qui a été tronqué par son passage à travers la fente en vis-à-vis de section rectangulaire. Ce faisceau est allongé à sa base (dans une section parallèle au plan de la fente), selon une direction qui correspond à la direction dans laquelle la fente est allongée.

Le capteur 20 assujetti au bras 16c est positionné en vis-à-vis du générateur 18. Il est apte, d'une part, à recevoir le rayonnement X provenant du générateur et ayant irradié l'objet (tête d'un patient) placé entre générateur et capteur et, d'autre part, à transformer ce rayonnement atténué par son passage à travers l'objet en un signal électrique représentatif d'une image radiographique de cet objet.

On notera que le capteur comprend une matrice de pixels qui est agencée en correspondance avec le faisceau issu de la fente de collimation.

Ce capteur est, par exemple, formé d'un scintillateur au phosphore comprenant la matrice de pixels à surface active et ses dimensions sont par exemple de 30 cm (hauteur) x 30 cm (largeur). Les pixels de la matrice ont par exemple une taille de 150 µm et forment ainsi une matrice de 2000 x 2000 pixels. Alternativement, le capteur est constitué d'une matrice de pixels à transfert de charge de type CCD de taille par exemple 5 cm x 5 cm et qui est munie d'une optique de focalisation avec un agrandissement optique de 6. Une électronique de commande et d'alimentation du capteur est prévue derrière celui-ci.

Les figures 2a et 2b illustrent des moyens de collimation 30 qui permettent de réaliser une fente de collimation à géométrie variable.

Des moyens de réglage sont prévus pour faire varier, sur commande, la géométrie de la fente et, notamment, son allongement selon deux directions perpendiculaires entre elles, par exemple horizontale et verticale.

Plus particulièrement, les moyens de réglage adaptés à modifier l'allongement de la fente selon une direction sont indépendants de ceux adaptés à modifier l'allongement dans l'autre direction, offrant ainsi une plus grande flexibilité de réglage.

Dans l'exemple illustré, l'appareil de radiologie comporte quatre moyens de réglage indépendants 50, 52, 54, 56 pour faire varier indépendamment la position de chacun des quatre bords 58, 60, 62, 64 définissant la fente de collimation.

Sur le support positionné devant la fenêtre de sortie de la Figure 1, sont successivement superposés l'agencement 30a de la figure 2b, puis celui 30b de la figure 2a.

Ces agencements n'ont pas été représentés de façon superposée par souci de clarté.

Plus particulièrement, l'agencement 30a de la figure 2a comporte deux bords 58, 60 de deux plaques 66, 68 disposées en vis-à-vis (par exemple rectangulaires) et qui sont fixées chacune respectivement à une autre plaque 70, 72 agencée perpendiculairement.

Chaque couple de plaques 66, 70 et 68, 72 forme ainsi un L ou un L tourné de 180°.

La seconde plaque 70, 72 de chaque couple est munie, sur un de ses bords qui est opposé à celui contre lequel est fixé la première plaque, d'une rangée longitudinale de dents 74, 76.

Un moyen de déplacement du bord 58 (respectivement 60) comporte un moteur 50 (respectivement 52) équipé sur son arbre de sortie d'un pignon denté 78 (respectivement 80). Ce pignon coopère avec les dents 76 (respectivement 74) pour provoquer le déplacement des plaques 72 et 68 dans la direction D1 selon l'un ou l'autre sens en fonction du sens de rotation du pignon.

Une lumière de guidage 82 (respectivement 84) est prévue dans la seconde plaque 72 (respectivement 70) et deux pions de guidage 86, 88 (respectivement 90, 92) solidaires du support précité sont positionnés dans cette rainure pour guider longitudinalement le déplacement de la plaque correspondante et donc du bord correspondant.

Cet agencement permet, en réglant l'écartement des bords opposés 58 et 60 selon la direction D1, d'ajuster l'une des dimensions de la fente et donc son allongement dans une direction.

De façon identique, l'agencement illustré 30b sur la figure 2b permet, en réglant l'écartement des bords opposés 62 et 64 selon la direction perpendiculaire D2, d'ajuster l'une des dimensions de la fente dans une autre direction.

Ainsi, en rapprochant les bords 62 et 64 et en écartant les bords 58 et 60 on conforme la fente de façon allongée selon la direction D1. On obtient alors une fente allongée suivant l'axe Z représentée à la figure 2c.

Au contraire, si les bords 62 et 64 sont écartés et les bords 58 et 60 rapprochés, la forme allongée de la fente est réalisée selon la direction D2. On obtient ainsi une fente allongée suivant un axe perpendiculaire à l'axe Z.

On peut également régler l'écartement des bords opposés 58, 60 et 62, 64 afin d'obtenir une fente de forme carrée ou proche d'une telle forme.

Les différents éléments représentés sur la figure 2b, à savoir les première et seconde plaques 100, 102 (respectivement 104, 106), les rainures 108 (respectivement 110), le moteur 54 (respectivement 56) et son pignon denté 116 (respectivement 118), ainsi que les pions de guidage 120, 122 (respectivement 124, 126) dans la lumière de guidage 128 (respectivement 130) sont identiques à ceux correspondants de la figure 2a mais sont seulement décalés de 90°.

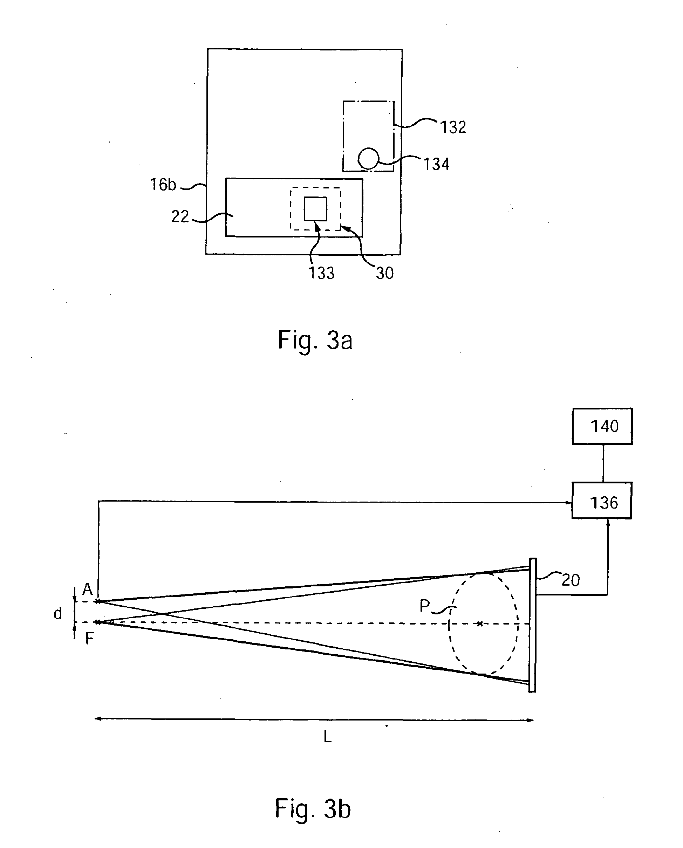

L'appareil de la figure 1 comprend également, comme représenté sur la figure 3a (vue de face du bras 16b), des moyens 132 d'acquisition d'au moins une image photographique de l'objet placé entre le générateur de rayons X et le capteur, à savoir la tête d'un patient.

Sur la figure 3a on a également représenté, en partie basse du bras 16b, le support 22 placé devant le générateur 18, les moyens de collimation 30 en pointillés et une fente de collimation 133 placée devant la fenêtre de sortie des rayons X.

Les moyens 132 qui prennent par exemple la forme d'un appareil photographique muni d'un objectif 134 sont positionnés le plus près possible du foyer d'émission des rayons X. Dans l'exemple de la figure 3a, les moyens 132 sont disposés au dessus du générateur et décalés latéralement par rapport à ce dernier. Toutefois, d'autres agencements sont envisageables, en fonction des contraintes d'environnement.

Ces moyens 132 sont également représentés sur la figure 3b par la lettre A, tandis que le foyer d'émission est représenté par la lettre F.

La distance d entre les moyens d'acquisition d'une image photographique et le foyer du générateur est petite par comparaison avec la distance L entre le foyer et le capteur 20.

A titre d'exemple, la distance d est égale à 5cm et la distance L est égale à 170 mm.

Ainsi, en positionnant les moyens d'acquisition d'image de façon aussi proche que possible du foyer du générateur, compte tenu de la place disponible autour des moyens de collimation placés devant la fenêtre d'émission des rayons X, on s'assure que l'angle sous lequel sont pris le ou les clichés photographiques de la tête du patient est très voisin de l'angle suivant lequel est émis le faisceau conique de rayons X vu du capteur.

A titre d'exemple, un écart de moins de 5 degrés fournit de bons résultats.

De ce fait, l'image photographique de la tête du patient et l'image céphalométrique sont superposables.

Comme illustré de façon très schématique sur la figure 3b, l'image photographique et l'image céphalométrique sont fournies respectivement par les moyens d'acquisition A et par le capteur 20 à une unité de traitement de données 136 incluant des moyens de mémorisation des images.

Un écran 140 de visualisation des images acquises individuellement et superposées est en outre relié à l'unité de traitement.

L'unité de traitement 136 et les moyens de visualisation 140 font partie de l'appareil de radiologie illustré à la figure 1.

L'unité de traitement 136 assure le contrôle du fonctionnement de l'appareil 10.

Cette unité peut être par exemple un ordinateur de type PC.

On notera que la matrice de pixels du capteur 20 présente des dimensions qui englobent les dimensions de la projection radiologique du faisceau de rayons X ayant irradié la tête P du patient.

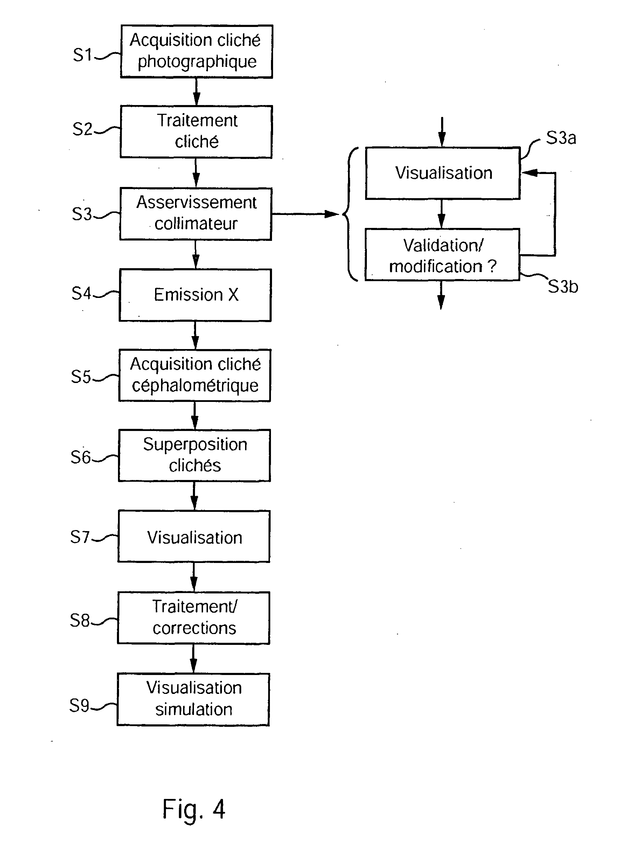

La figure 4 représente un algorithme détaillant les principales étapes d'un procédé selon l'invention et qui peut être mis en oeuvre par exemple par l'appareil 10.

Cet algorithme est par exemple mémorisé dans une zone mémoire de l'unité de traitement 136 et est exécuté sur commande.

Pour la mise en oeuvre du procédé selon l'invention, on considère qu'un patient est positionné entre le générateur de rayons X et le capteur illustrés sur la figure 1 et que sa tête est immobilisée à proximité du capteur, c'est-à-dire à grande distance du générateur, par exemple à environ 150 cm.

Lorsque le praticien met en oeuvre l'appareil, par exemple à partir d'un clavier et d'un organe d'interaction tel qu'une souris, non représentés sur les figures mais qui interagissent avec l'unité de traitement 136 et l'écran 140 de la figure 3b, un mode d'acquisition d'un ou de plusieurs clichés photographiques de la tête du patient (vue de face ou de profil) est déclenché lors d'une première étape S1.

Ce ou ces clichés sont mémorisés.

Au cours d'une deuxième étape S2, un traitement du cliché est prévu afin de ne conserver que les contours de la tête du patient.

En effet, ces seuls contours sont suffisants pour fournir l'information nécessaire à l'utilisateur de l'appareil.

Ce traitement est effectué par l'unité de traitement 136 qui détermine en outre les dimensions du contour de la tête.

L'algorithme comporte une troisième étape S3 d'asservissement des moyens de collimation en fonction du ou des clichés photographiques acquis à l'étape S1 et, notamment, des dimensions du contour de la tête du patient obtenues à l'étape S2.

On s'assure ainsi que les dimensions du faisceau de rayons X émis par le foyer F et collimaté par les moyens de collimation soient ajustées aux dimensions du contour de la tête du patient.

Ainsi, le ou les clichés céphalométriques qui vont être réalisés par l'appareil seront parfaitement adaptés aux dimensions de la tête du patient.

De ce fait, la tête du patient ne sera pas tronquée sur le ou les clichés et le patient n'aura pas reçu de doses de rayonnement inutiles comme c'était le cas par le passé.

D'un point de vue pratique, l'asservissement des moyens de collimation consiste à régler les dimensions du faisceau de rayons X de façon adaptée aux dimensions du contour de la tête du patient.

Ce réglage comprend plus particulièrement le réglage de l'allongement de la fente de collimation des moyens 30a et 30b illustrés sur les figures 2a et 2b, selon des directions perpendiculaires entre elles. Ce réglage est commandé par l'unité de traitement 136 de la figure 3b à partir des dimensions du contour de la tête calculées à l'étape S2.

De façon optionnelle, l'algorithme peut comprendre une étape S3a de visualisation sur l'écran 140 des bords de la fente du collimateur projetés une fois ajustés aux dimensions du contour de la tête, et qui sont superposés au contour de la tête.

Cette étape de visualisation permet de s'assurer que l'asservissement du collimateur à la tête du patient est correct et peut être validé au cours d'une étape S3b.

Dans l'hypothèse où l'écartement des bords de la fente de collimation ne serait pas adapté aux dimensions du contour de la tête du patient, soit parce que cet écartement serait trop grand ou trop petit par rapport à la tête, l'étape S3b prévoit également de modifier le réglage de la fente de collimation afin d'obtenir un ajustement par rapport au contour de la tête du patient. Alternativement, l'étape S3b permet de revenir à des fenêtres de collimation préprogrammées par défaut.

L'étape S3b est alors suivie de l'étape S3a de visualisation afin que l'utilisateur de l'appareil puisse voir le nouveau réglage auquel il a procédé.

Ensuite, l'étape S3b est à nouveau effectuée pour que l'utilisateur puisse valider le réglage.

L'algorithme comporte ensuite une étape S4 d'émission d'un faisceau conique de rayons X, ce faisceau étant collimaté par la fente de collimation dont le réglage a été obtenu et validé à l'étape S3.

Ainsi, le faisceau collimaté est parfaitement adapté aux dimensions du contour de la tête du patient.

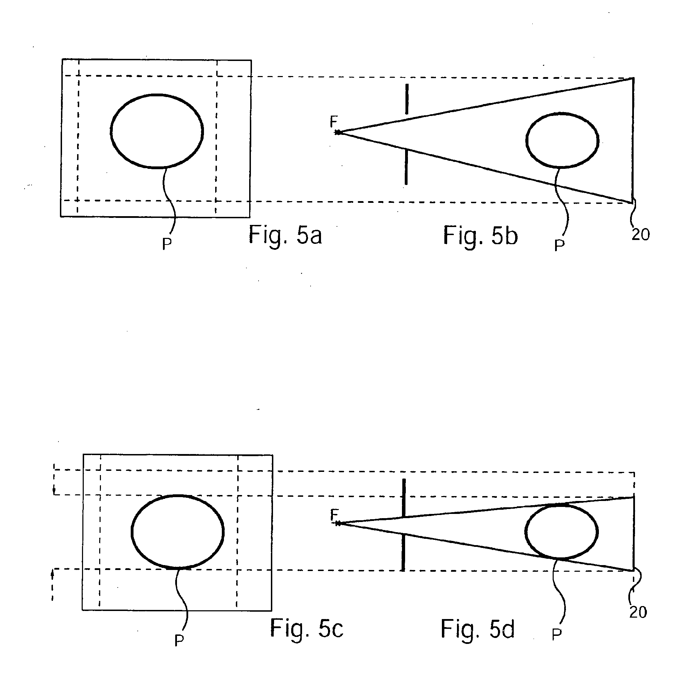

La figure 5a illustre la visualisation sur l'écran de la tête du patient P et de la projection conique des bords de la fente lorsque l'asservissement selon l'invention n'a pas été effectué.

La figure 5b illustre la position correspondante des bords de la fente, de la tête et du capteur dans l'agencement présenté à la figure 3b.

Dans une telle position le patient recevrait une surdose de rayonnement.

La figure 5c illustre la visualisation sur l'écran de la tête P, de la projection conique des bords de la fente après asservissement en fonction du cliché photographique précédemment réalisé.

Les moyens de collimation sont représentés sur la figure 5d dans une position ajustée obtenue grâce à l'asservissement.

La configuration du faisceau de rayons X est ainsi adaptée à la tête du patient et ce dernier reçoit ainsi une dose de rayonnement optimisée. La projection radiologique du faisceau qui a irradié la tête est inscrite dans la surface active du capteur 20.

L'étape S5 prévoit d'acquérir un ou plusieurs clichés céphalométriques de la tête du patient. Ce ou ces clichés sont acquis de façon instantanée afin que le patient ne bouge pas, évitant ainsi des distorsions. On notera qu'un cliché est par exemple réalisé en ½ s.

Dans l'exemple de réalisation décrit, les clichés photographiques et céphalométriques correspondent à des vues de profil de la tête du patient.

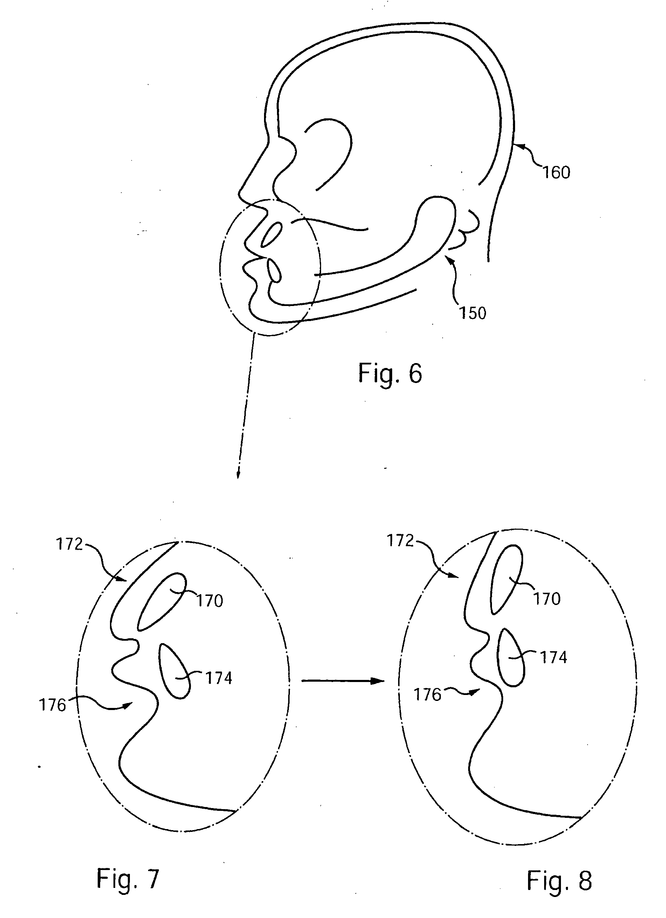

Après acquisition d'un cliché céphalométrique 150, il est prévu au cours d'une étape S6 de superposer le cliché céphalométrique 150 et le cliché photographique 160 comme illustré sur la figure 6.

Le cliché photographique 160 fait ainsi apparaître les tissus mous de la tête du patient (nez, lèvres, menton ...), tandis que le cliché céphalométrique 150 fait apparaître les tissus durs (os, dents ...).

On notera que la superposition des clichés est réalisée de façon particulièrement fiable et précise grâce au positionnement des moyens d'acquisition du cliché photographique à très faible distance du foyer d'émission des rayons X.

Il convient de noter que la superposition du cliché photographique sur l'image céphalométrique nécessite d'appliquer une transformation géométrique basée sur la reconnaissance de profil et de point cible pour obtenir une parfaite correspondance. On peut par exemple utiliser à cet effet les branches de maintien du patient du dispositif 21 de la figure 1.

Comme déjà mentionné, vus du capteur 20 illustré sur la figure 3b, les deux clichés photographique et céphalométrique peuvent être considérés avoir été pris avec le même angle de prise de vue.

Une correspondance quasi parfaite entre les deux clichés permet de positionner correctement sur la même vue (Figure 6) les tissus mous et les tissus durs les uns par rapport aux autres.

Cette superposition des clichés est suivie de l'étape S7 de visualisation des clichés ainsi superposés qui est illustrée sur la figure 6 déjà mentionnée.

Cette visualisation est par exemple effectuée sur l'écran 140 de la figure 3b.

L'image ainsi obtenue sur l'écran de visualisation permet au praticien, par exemple un orthodontiste, d'établir un diagnostic en identifiant certains défauts à corriger par exemple dans la mâchoire du patient.

Il peut ainsi déterminer les corrections qui sont à apporter à cette mâchoire ainsi que le traitement approprié.

Dans l'exemple illustré sur la figure 6 et représenté de façon agrandie sur la figure 7, l'implantation de l'incisive 170 de la mâchoire du patient est telle que celle-ci est particulièrement inclinée par rapport à la verticale vers l'avant de la bouche du patient, ce qui provoque une déformation de la lèvre supérieure 172.

De même, la dent 174 est implantée dans la mâchoire inférieure de façon telle qu'elle est fortement inclinée par rapport à la verticale en direction de l'avant de la bouche, ce qui provoque là aussi une déformation de la lèvre inférieure 176.

Partant de ce constat, le praticien prend des mesures et éventuellement réalise des tracés afin de déterminer les corrections à apporter à la mâchoire du patient, ainsi que le traitement approprié (par exemple, port d'un appareillage afin de rectifier le positionnement des dents 170 et 174).

Cette étape correspond à l'étape S8 de l'algorithme.

L'étape suivante S9 permet de visualiser, de façon prévisionnelle, les effets au cours du temps du traitement préconisé par le praticien sur la mâchoire du patient.

La déformation des tissus durs du cliché céphalométrique est obtenue par calcul, à partir des données sélectionnées par le praticien et lorsqu'il a déterminé les corrections à apporter et le traitement approprié.

Cette étape est effectuée par l'unité de traitement 136 et correspond à l'exécution d'un algorithme connu en soi et disponible sur le marché. Il s'agit par exemple d'un logiciel commercialisé par la société Practice Works.

Les effets ainsi simulés sur les tissus durs du cliché céphalométrique sont illustrés à la figure 8.

De même, la façon dont les tissus mous (les lèvres en particulier) sont déformés de façon correspondante au cours du temps, est obtenue grâce à un algorithme de morphing, connu en soi, qui est également mis en oeuvre par l'unité de traitement 136.

La visualisation des effets ainsi simulés sur les tissus mous du patient est également illustrée sur la figure 8 correspondant à la superposition des deux clichés, après déformation de chacun d'eux.

On notera que la déformation des tissus durs du cliché céphalométrique et la déformation des tissus mous du cliché photographique sont réalisés de façon indépendante l'une de l'autre dans la mesure où, dans l'image de la figure 6 illustrant la superposition des deux clichés, chacun d'eux correspond à un ensemble de données distinctes et donc traitables de façon séparée.

Grâce à la visualisation de cette simulation, le praticien, de même que le patient, sont à même d'apprécier les impacts du traitement préconisé par le praticien, d'une façon particulièrement réaliste.

## Revendications

1. Appareil de radiologie dentaire comprenant :
- un générateur de rayonnement X (18) adapté à générer un faisceau de rayons X en direction d'une tête d'un patient,
- des moyens de collimation (30) adaptés à conférer au faisceau de rayons X généré des dimensions données;
- un capteur (20) disposé en vis-à-vis du générateur, recevant la projection radiologique du faisceau collimaté ayant irradié la tête du patient et fournissant une image céphalométrique de la tête du patient,
**caractérisé en ce que** l'appareil comporte :
- des moyens (132) d'acquisition d'au moins une image photographique de la tête du patient,
- des moyens d'asservissement (136) des moyens de collimation en fonction de ladite au moins une image photographique afin que les dimensions du faisceau de rayons X collimaté soient ajustées aux dimensions de la tête du patient.

2. Appareil selon la revendication 1, **caractérisé en ce que** le capteur (20) est un capteur surfacique à matrice de pixels présentant des dimensions qui englobent les dimensions de la projection radiologique du faisceau ayant irradié la tête du patient.

3. Appareil selon la revendication 1 ou 2, **caractérisé en ce que** le générateur de rayonnement X (18) comprend un foyer d'émission des rayons X, les moyens (132) d'acquisition d'au moins une image photographique étant positionnés le plus près possible du foyer.

4. Appareil selon l'une des revendications 1 à 3, **caractérisé en ce que** les moyens de collimation (30) comprennent un collimateur à fente réglable.

5. Appareil selon la revendication 4, **caractérisé en ce que** le collimateur à fente réglable comporte des moyens de réglage de l'allongement de la fente selon des directions perpendiculaires entre elles.

6. Appareil selon la revendication 5, **caractérisé en ce que** les moyens de réglage sont indépendants selon les directions.

7. Appareil selon l'une des revendications 4 à 6, **caractérisé en ce que** la fente réglable est délimitée par quatre bords qui sont déplaçables de façon indépendante l'un de l'autre.

8. Appareil selon l'une des revendications 1 à 7, **caractérisé en ce qu'**il comprend des moyens d'obtention du contour de la tête du patient à partir de ladite au moins une image photographique acquise.

9. Appareil selon la revendication 8, **caractérisé en ce que** les moyens d'asservissement sont adaptés à asservir les moyens de collimation en fonction des dimensions du contour de la tête du patient afin que les dimensions du faisceau de rayons X collimaté soient ajustées aux dimensions du contour.

10. Procédé pour produire une image céphalométrique de la tête d'un patient comprenant les étapes suivantes :
- génération (S4) par un générateur de rayonnement X (18) d'un faisceau de rayons X en direction de la tête d'un patient,
- collimation du faisceau de rayons X généré afin de lui conférer des dimensions données,
- réception par un capteur (20) en vis-à-vis de la projection radiologique du faisceau collimaté ayant irradié la tête du patient,
- fourniture d'une image céphalométrique à partir de la projection radiologique reçue,
**caractérisé en ce que** le procédé comporte en outre les étapes suivantes :
- acquisition (S1) d'au moins une image photographique de la tête du patient,
- asservissement (S3) de la collimation du faisceau de rayons X en fonction de ladite au moins une image photographique afin que les dimensions du faisceau de rayons X collimaté soient ajustées aux dimensions de la tête du patient.

11. Procédé selon la revendication 10, **caractérisé en ce que** le capteur (20) est un capteur surfacique à matrice de pixels présentant des dimensions qui englobent les dimensions de la projection radiologique du faisceau ayant irradié la tête du patient, l'acquisition de la projection radiologique étant effectuée de façon instantanée.

12. Procédé selon la revendication 10 ou 11, **caractérisé en ce que** le générateur de rayonnement X (18) comprend un foyer d'émission des rayons X, l'acquisition de ladite au moins une image photographique étant effectuée à partir d'un emplacement qui est le plus proche possible du foyer.

13. Procédé selon l'une des revendications 10 à 12, **caractérisé en ce que** l'étape (S3) d'asservissement du faisceau de rayons X en fonction de ladite au moins une image photographique comprend le réglage des dimensions du faisceau.

14. Procédé selon la revendication 13, **caractérisé en ce que** le réglage des dimensions du faisceau comprend plus particulièrement le réglage de l'allongement d'une fente de collimation selon des directions perpendiculaires entre elles.

15. Procédé selon l'une des revendications 10 à 14, **caractérisé en ce qu'**il comprend une étape (S2) d'obtention du contour de la tête du patient à partir de ladite au moins une image photographique acquise.

## Patentansprüche

1. Eine Dentalröntgeneinheit, die Folgendes aufweist:
einen Röntgengenerator, der ausgelegt ist, um einen Röntgenstrahl in der Richtung eines Kopfes eines Patienten zu erzeugen;
Kollimationsmittel, die ausgelegt sind, um gegebene Abmessungen für den erzeugten Röntgenstrahl vorzusehen, um einen kollimierten Röntgenstrahl zu erzeugen;
einen Sensor, der auf den Generator ausgerichtet ist, um die Röntgenprojektion des kollimierten Strahls aufzunehmen, der den Kopf des Patienten bestrahlt, um ein cephalometrisches Abbild des Kopfes des Patienten zu erzeugen;
Mittel zum Erfassen wenigstens eines photographischen Abbildes des Kopfes des Patienten; und
Mittel zum automatischen Steuern der Kollimationsmittel gemäß dem wenigstens einen photographischen Abbild, so dass die gegebenen Abmessungen des kollimierten Röntgenstrahls an die Abmessungen des Kopfes des Patienten angepasst werden.

2. Dentalröntgeneinheit nach Anspruch 1, wobei der Sensor ein Pixelmatrix-oberflächensensor mit Abmessungen ist, die die Abmessungen der Röntgenstrahlprojektion umfassen, die den Kopf des Patienten bestrahlt hat.

3. Dentalröntgeneinheit gemäß Anspruch 1, wobei der Röntgengenerator eine Röntgenemissionskammer aufweist, und die Mittel zum Erfassen wenigstens eines photographischen Abbildes benachbart zu der Kammer angeordnet sind.

4. Dentalröntgeneinheit nach Anspruch 1, wobei die Kollimationsmittel einen Kollimator mit einstellbarem Schlitz aufweisen.

5. Dentalröntgeneinheit nach Anspruch 4, wobei der Kollimator mit einstellbarem Schlitz Mittel zum Einstellen der Länge des Schlitzes senkrecht zwischen ihnen aufweist.

6. Dentalröntgeneinheit nach Anspruch 5, wobei die Mittel zum Einstellen richtungsunabhängig sind.

7. Dentalröntgeneinheit nach Anspruch 4, wobei der Schlitz durch vier Kanten begrenzt wird, die sich auf eine Weise verschieben, die unabhängig voneinander ist.

8. Dentalröntgeneinheit nach Anspruch 1, die weiter Mittel zum Erlangen des Umrisses des Kopfes des Patienten aufweist, von dem wenigstens ein photographisches Abbild erfasst wird.

9. Dentalröntgeneinheit nach Anspruch 8, wobei die Mittel zur automatischen Steuerung ausgelegt sind, um automatisch die Kollimationsmittel als eine Funktion der Abmessungen des Umrisses des Kopfes des Patienten zu steuern, wobei die Abmessungen des kollimierten Röntgenstrahls an die Abmessungen des Umrisses angepasst werden.

10. Ein Verfahren zum Erzeugen eines cephalometrisches Abbildes des Kopfes eines Patienten, das Folgendes aufweist:
Erzeugen, durch einen Röntgengenerator, eines Röntgenstrahls in der Richtung des Kopfes eines Patienten;
Kollimieren des erzeugten Röntgenstrahls, um die gegebenen Abmessungen zu übertragen;
Leiten des kollimierten Strahls, der den Kopf des Patienten bestrahlt hat, in Richtung eines Sensors, der zur radiologischen Projektion weist;
Erzeugen eines cephalometrischen Abbildes aus der aufgenommenen radiologischen Projektion;
Akquirieren wenigstens eines photographischen Abbildes des Kopfes des Patienten; und
automatisches Steuern der Kollimation des Röntgenstrahls gemäß dem wenigstens einen photographischen Abbild, so dass die Abmessungen des kollimierten Röntgenstrahls auf die Abmessungen des Kopfes des Patienten angepasst werden.

11. Verfahren nach Anspruch 10, wobei der Sensor ein Pixelmatrixoberflächensensor mit Abmessungen ist, die die Abmessungen der Röntgenstrahlprojektion umfassen, die den Kopf des Patienten bestrahlt hat und wobei das Erfassen der radiologischen Projektion sofort ausgeführt wird.

12. Verfahren nach Anspruch 10, wobei der Röntgengenerator eine Röntgenemissionskammer aufweist; und die Erfassung wenigstens eines photographischen Abbildes an einem Ort durchgeführt wird, der so nah wie möglich an der Kammer ist.

13. Verfahren nach Anspruch 10, wobei der automatische Steuerungsschritt des Röntgenstrahls als eine Funktion des wenigstens eines photographischen Abbildes die Anpassung der Abmessungen des Strahls beinhaltet.

14. Verfahren nach Anspruch 13, wobei die Anpassung der Abmessungen des Strahls Mittel zum Anpassen der Länge des Kollimationsschlitzes senkrecht zwischen ihnen aufweist.

15. Verfahren nach Anspruch 10, das weiter Vorsehen eines Schrittes aufweist, um den Umriss des Kopfes des Patienten zu erlangen, von dem wenigstens ein photographisches Abbild erfasst wird.

## Claims

1. A dental x-ray unit comprising:
an X-ray generator adapted to generate an X-ray beam in the direction of a patient's head;
a means of collimation adapted to provide given dimensions to the generated X-ray beam to generate a collimated X-ray beam;
a sensor, disposed facing the generator, receiving the x-ray projection of the collimated beam irradiating the patient's head to generate a cephalometric image of the patient's head;
means to acquire at least one photographic image of the patient's head; and
means of automatic control of the means of collimation according to the at least one photographic image so that the given dimensions of the collimated X-ray beam are adjusted to dimensions of the patient's head.

2. The dental x-ray unit according to Claim 1, wherein the sensor is a pixel matrix surface sensor with dimensions that encompass the dimensions of the X-ray beam projection that irradiated the patient's head.

3. The dental x-ray unit according to Claim 1, wherein the X-ray generator includes an X-ray emission chamber, and the means to acquire at least one photographic image is positioned adjacent the chamber.

4. The dental x-ray unit according to Claim 1, wherein the means of collimation includes collimator having an adjustable slot.

5. The dental x-ray unit according to Claim 4, wherein the adjustable slot collimator comprises means to adjust the length of the slot perpendicularly between them.

6. The dental x-ray unit according to Claim 5, wherein the means of adjust is independent directionally.

7. The dental x-ray unit according to Claim 4, wherein the slot is delimited by four edges that slide in a manner that is independent from each other.

8. The dental x-ray unit according to Claim 1, further comprising means to obtain the outline of the patient's head from which the at least one photographic image is acquired.

9. The dental x-ray unit according to Claim 8, wherein the means of automatic control is adapted to automatically control the means of collimation in function of the dimensions of the outline of the patient's head wherein the dimensions of the collimated X-ray beam are adjusted to the dimensions of the outline.

10. A method to produce a cephalometric image of the head of a patient, comprising:
generating, by an X-ray generator, an X-ray beam in the direction of a patient's head;
collimating the generated X-ray beam to confer to it given dimensions;
directing the collimated beam which irradiated the patient's head toward a sensor facing the radiological projection;
generating a cephalometric image from the received radiological projection;
acquiring at least one photographic image of the patient's head; and
automatically controlling the collimation of the X-ray beam according to the at least one photographic image so that the dimensions of the collimated X-ray beam are adjusted to dimensions of the patient's head.

11. The method according to Claim 10, wherein the sensor is a pixel matrix surface sensor with dimensions that encompass the dimensions of the x-ray beam projection that irradiated the patient's head; and the acquisition of the radiological projection is performed instantly.

12. The method according to Claim 10, wherein the X-ray generator comprises an x-ray emission chamber; and the acquisition of at least one photographic image is performed from a location that is as close as possible to the chamber.

13. The method according to Claim 10, wherein the automatic control stage of the X-ray beam as a function of the at least one photographic image includes the adjustment of the dimensions of the beam.

14. The method according to Claim 13, wherein the adjustment of the dimensions of the beam comprises means to adjust the length of the collimation slot perpendicularly between them.

15. The method according to Claim 10, further comprises providing a stage to obtain the outline of the patient's head from which the at least one photographic image is acquired.
